# EUROPEAN PATENT SPECIFICATION

(11) **EP 2 731 575 B1**
(45) Date of publication and mention of the grant of the patent: **09.10.2024**
(21) Application number: 11869290.4
(22) Date of filing: 02.08.2011
(51) Int. Cl.: A61J 1/06, A61M 3/00, A61H 35/04, A61M 31/00, B65D 1/09, A61M 3/02

(54) **PORTABLE AMPOULE WITH A SPECIALIZED TIP AND SEALER**
TRAGBARE AMPULLE MIT EINER SPEZIELLEN SPITZE UND EINEM VERSCHLUSS
AMPOULE PORTATIVE COMPRENANT UNE POINTE SPÉCIALISÉE ET UN OBTURANT

(30) Priority: 14.07.2011 US 201113183387
(43) Date of publication of application: 21.05.2014
(73) Proprietor: Mehta, Ketan C., Santa Rosa, California 95403 (US)
(72) Inventor: Mehta, Ketan C., Santa Rosa, California 95403 (US)
(74) Representative: Boult Wade Tennant LLP
(86) International application number: PCT/US2011/046221
(87) International publication number: WO 2013/009327

(56) References cited:
- WO-A1-2010/051218
- WO-A1-83/01052
- FR-A1- 2 854 574
- US-A- 5 116 311
- US-A- 5 908 124
- US-A1- 2002 100 741
- US-A1- 2003 146 245
- US-A1- 2008 228 162
- US-B2- 7 810 680

## Description

### Technical Field

The subject matter of this application is generally related to dispensers.

### Background

People in many parts of the world perform nasal cleansing (or nasal irrigation) using a neti pots or other products on a routine basis. Nasal cleansing is also incorporated into some forms of yoga practice, such as in Jala neti. Jala neti is a Sanskrit term that refers to cleansing and means "water cleansing". The solution for rinsing the nasal passages can be a saline solution. Some patients use nasal rinsing to reduce allergies, improve breathing, eliminate post-nasal drip or sinus infections, moisten dry nasal passages, avoid catching a cold, or otherwise generally improve one's health. Other uses are possible. Conventional nasal rinse products, however, are bulky and do not fit within purses, backpacks, briefcases, or other personal items that are carried around.

WO 83/01052 describes a container with a wall that is integrated with a cap having a plug that extends sufficiently through a weakened region of the container wall to prevent inadvertent rupture of the cap during shipment.

US 2008/228162 describes a single-use, blow-moulded container for medical or pharmaceutical use.

WO 2010/051218 describes a container comprising a contiguous piece having: a hollow body having a neck, which terminates at a mouth, a breakable transition portion being contiguously coupled to the neck and a handle portion coupled to the transition portion at one end and having a closure member at the other end.

US 2002/100741 describes a hermetically sealed container including a dispensing nozzle and a hollow closure with a lobate region removeably secured along a frangible web.

US 2003/146245 describes a hermetically sealed container having a closure connected to the container by a frangible web.

FR 2854574 describes an instrument comprising a reservoir that terminates in a nozzle that is blunt-conical or in the form of an olive.

US 5,116,311 provides a method for administration of pharmacological solutions into the nasal cavity whereby the patient's head is being bent forward 55-90° from an upright position as well as a device for use in the administration. The device fits to a nostril at one end and at the other end consists of a container may be prefilled with the solution that is to be administered. The upper part of the device, the olive, that fits in the nostril is conical and has an opening having a diameter is between 3-10 mm to avoid spraying or jet streaming the contents of the device into the nasal cavity. The opening is unsealed. The diameter of the base of the olive is 15-23 mm to provide proper tightening of all sizes of nostrils.

### Summary

In accordance with the present invention there is provided a dispensing device, as described in claim 1.

The present disclosure relates to a portable ampoule with a specialized tip and sealer. According to the invention, the portable ampoule for dispensing fluid includes a body configured to contain cleansing solution. A neck is coupled to the body and configured to control the flow of the solution. A tip is coupled to the neck and has an aperture for solution release. A sealing device is coupled to the tip and configured to seal the aperture. The sealing device permanently unseals the aperture upon decoupling from the tip. The tip includes a tapered surface that permits the tip to conform to nostrils of different sizes, the exterior of the tip being tapered outwardly from a wide portion up near the bottom of the tip to a narrow portion near the top of the tip, and the tip being sized to prevent the wide portion from extending all the way into the user's nostril. The bottom diameter of tip is between 10 and 20 millimeters, and the top diameter of tip is between 3 and 8 millimeters. The sealing device includes an opener having a twist coupler to facilitate twisting motion for removing the sealing device to unseal the aperture.

Examples useful for understanding the invention may include one or more of the following features. The sealing device may be for a single use such that upon removing the sealing device from the tip, the sealing device is permanently displaced from the tip. The opener may be a planar holding structure to accommodate application of moment or torque to the twist coupler. The opener may include an outer rim to provide torque support and an inner rim to secure the twist coupler to the outer rim. The sealing device may include a sealer and the twist coupler may be coupled to the tip through the sealer.

The body may include a rib structure to facilitate holding or gripping of the body to avoid slipping motion generated when the body is rotated in an opposite direction with respect to the sealing device. The rib structure may be flush or contiguous with edges of the opener. The rib structure may extend from a bottom portion of the tip to a bottom portion of the body, wherein a bottom surface of the opener body aligns with the bottom portion of the tip. The twist coupler includes inner side walls that conform with but do not contact sidewalls of the tip.

The tip may be further configured to attenuate the pressure of solution stored in the body and facilitate dispensing of the solution with sufficient pressure to deliver the solution to nasal tissue without displacing the nasal tissue. The neck may have a diameter smaller than a diameter of the body to facilitate a dispensing speed of the solution. The tip may be conically shaped with a convex curved surface tapered from a surface on which the aperture is formed to a bottom portion of the tip. The bottom portion may include a diameter with sufficient dimension to prevent the tip from extending into a user's nostril. The bottom portion of the tip may include rounded or chamfered edges. The tip may include a tapered surface that conforms to nostrils of different sizes.

The details of one or more implementations of the subject matter described in this specification are set forth in the accompanying drawings and the description below. Other features, aspects, and advantages of the subject matter will become apparent from the description, the drawings, and the claims.

### BRIEF DESCRIPTION OF DRAWINGS

FIG. 1 A is a schematic front view of an ampoule.
FIG. 1B is a schematic top view of the ampoule.
FIG. 2 is a schematic side view of the ampoule.
FIG. 3 is a schematic cross-sectional view at A-A of the ampoule.
FIGS. 4A and 4B are schematic views of the ampoule before and after opening respectively.
FIG. 5A is a schematic view of a second ampoule example useful for understanding the invention.
FIG. 5B is a schematic cross-sectional view along the longitudinal axis of the second ampoule example that is useful for understanding the invention.

Like reference symbols in the various drawings indicate like elements.

### DETAILED DESCRIPTION

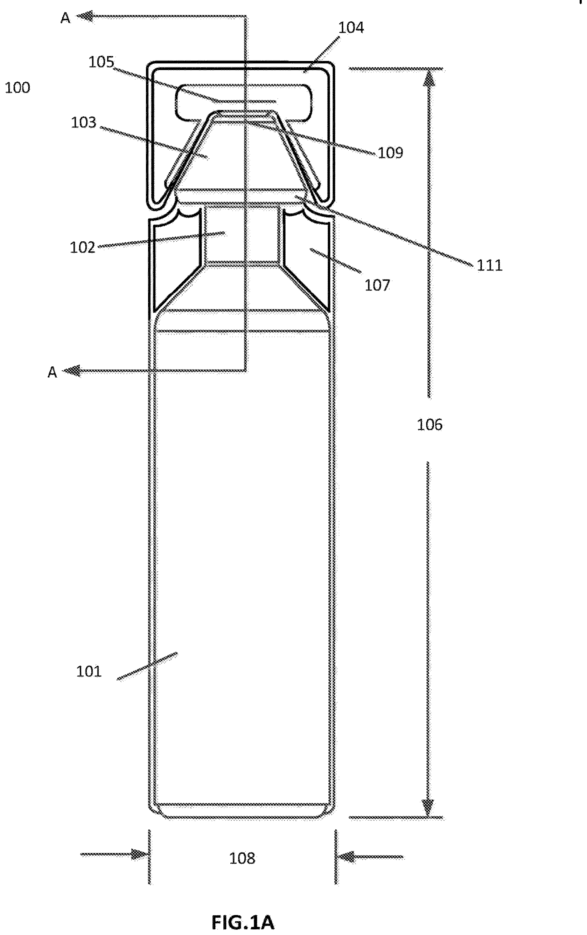

FIG 1A is a schematic front view of an ampoule 100. As shown in FIG 1A, the ampoule 100 includes a body 101, a neck 102, a tip 103 and an opener 104. The neck 102 connects the body 101 to the tip 103. The opener 104 allows users to twist open the tip 103 at a twist coupler 105. The body 101 can be, for example, a container of saline solution or any other fluid suitable for irrigating cavities (e.g. nasal cavities). The ampoule 100 can be used, for example, to provide nasal rinsing (or irrigation or nasal lavage), such as to treat allergies, improve breathing, eliminate post-nasal drip or sinus infections, moisten dry nasal passages, etc. In some implementations, the tip 103 can attenuate the pressure of fluid stored in the body 101, dispensing fluid at a gentle pressure. The gentle pressure can be sufficient to deliver a flow of fluid to tissue without the pressure being so great as to displace the tissue.

The body 101 is a fluid container (e.g. a bottle, can or other container) that securely stores fluid and allows users to apply pressure (e.g. to the container) to expel the stored fluid. For example, the body 101 can be made of thermoplastic polymers, thermosetting polymers, or any other appropriate materials that allows for deformation in order to pressurize the bottle for fluid release. In some implementations, the ampoule 100 can be pressurized for maintaining shape during transportation. The body 101 is a cylindrical shape and may be a cylindrical shape of a uniform diameter. In other examples, the diameter can vary along its longitudinal axis, for example, a tapered shape, a curved shape, a diamond shape, or other shapes. The body 101 can be a thin-walled structure of uniform thickness and/or variable thickness for functional requirements. For example, to facilitate deformation, some locations on the body 101 can be thinner than the rest. As another example, other locations on the body 101 can be thicker for structural reinforcement, such as a portion at or near the bottom of the ampoule 100. Grooves or depressions can be included in the body to facilitate gripping by the human hand.

In some implementations, the dimension of the neck 102 can be tailored to accommodate an increase output flow velocity of fluid from the body 101. The neck 102 can be made of the same material as the body 101, such as thermoplastic polymers, thermosetting polymers, etc. In some implementations, the neck 102 is a cylindrical shape of a uniform diameter that is significantly smaller than that of the body 101 (e.g., the diameter of neck 102 is 50% smaller than the maximum diameter of the body 101). Other ratios between the diameter of the neck 102 and that of the body 101 are also contemplated. In some implementations, a small neck diameter allows the output flow velocity to increase (e.g., because for a given amount of fluid volume that is displaced, the narrower the cross-sectional area of a passage, the higher the flow velocity). The neck 102 can also be a thin-walled structure of uniform thickness and/or variable thickness for functional requirements. For example, at the location where the body 101 and the tip 103 intersects, extra wall thickness can be implemented to enhance structural integrity.

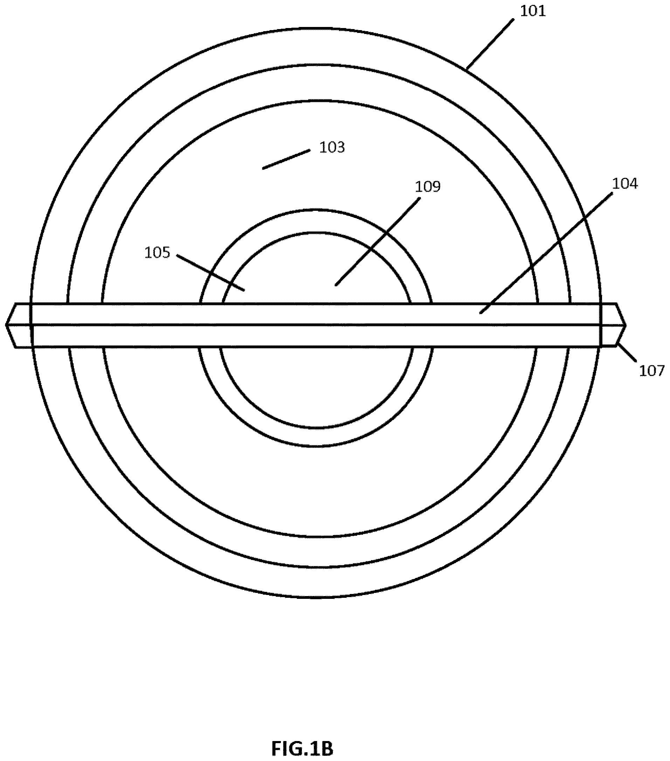

As shown in FIG. 1, the tip 103 is connected to the neck 102, which is partially encapsulated by the twist coupler 105. An aperture 109 is revealed upon removing the twist coupler 105. In some implementations, the tip 103 can be conically shaped with a convex curved surface leading from the aperture 109 toward the bottom portion 111 of the tip 103. In some implementations, the tip 103 can be gumdrop- or mushroom-shaped. The tip 103 may include rounded edges such as an upper rounded edge and a lower rounded edge. The rounded edges may have a substantially large rounding radius to facilitate manufacturing process and avoid causing discomfort to user during use and/or handling. For example, the upper rounded edge can be of a rounding radius between 0.5 to 4 mm, such as 1 mm. This allows the tip 103 to comfortably contact with the user at various insertion angles without excessive friction to cause irritation or discomfort. The lower rounded edge may have a rounding radius between 1 to 6 mm, such as 2 mm. This allows the tip 103 to be safely completely enclosed by a user's nostril and cause minimum friction and discomfort during removal. This also allows the tip 103 to avoid stress concentration during production, transportation and use.

The tip 103 includes a tapered surface that permits the tip 103 to conform to nostrils of different sizes. Specifically, the exterior of the tip 103 is tapered outwardly. The tip 103 tapers from a wide portion up (e.g., the portion near the bottom of the tip 103) to a narrow portion (e.g., the portion near the top of the tip 103). The tip 103 is sized to prevent the wide portion from extending all the way into a user's nostril. In some implementations, the transition from the end of the wide portion to the neck 102 can be rounded or chamfered to avoid any sharp edges. The tip 103 can be made of the same material as the body 101, such as thermoplastic polymers, thermosetting polymers, or other suitable materials tailored for human use. The tip 103 can be a thin-walled structure of approximate uniform thickness.

In some implementations, the twist coupler 105 is breakably coupled to the tip 103 at the aperture 109 and reinforceably affixed to the opener 104. The opener 104 can be sized to facilitate the twisting motion for twist opening the twist coupler 105. In some implementations, the twist coupler 105 acts as a one-time seal to the tip 103 so that upon twist opening the twist coupler 105, the twist coupler 105 is permanently displaced from the tip 103. In so doing, the tip 103 cannot re-seal the aperture 109, and the ampoule 100 can be discarded after one-time use.

In some implementations, the twist coupler 105 is a thin-walled structure coupled to the tip 103 by, for example, heated compression or any similar techniques, sealingly adhering two adjacent walls that can be broken with a twisting motion when the shear stress exceeds the bonding strength between the two thin walls. The twist coupler 105 can be of a donut shape, a tire shape, or any other appropriate shape to encapsulate the aperture 109. The twist coupler 105 can be made of the same material as the tip 103, such as thermoplastic polymers, thermosetting polymers, and other suitable materials.

In some implementations, the opener 104 is integrally and reinforceablly affixed to the twist coupler 105. The opener 104 serves as a holding structure for user's fingers to apply a moment/torque to the twist coupler 105. In some implementations, the opener 104 is a plane structure of a thickness that defies significant bending deformation under normal use. The opener 104 may include an outer rim to provide torque support and an inner rim to secure the twist coupler to the outer rim. For example, the outer rim may be of a thicker thickness than the inner rim so that when a torque is applied to the outer rim, structural deformation is limited by the material strength of the outer rim. The thickness of the outer rim may be between about 1 and 4 mm, such as 2.5 mm. The primary function of the inner rim is to secure the twist coupler 105 to the outer rim. As the inner rim deforms under loading, tensile stress can become the major stress within the component to provide a transmitting force to rotate the twist coupler 105. Therefore, the thickness of the inner rim may not require a large thickness, between about 0.5 to 2 mm, such as 1 mm. This also saves production material and reduces portable weight of the ampoule 100.

In some examples useful for understanding the invention, the opener 104 is affixed to a cap (not shown) instead of the twist coupler 105 for re-useable purposes. The cap may be a screw type cap that has spiral rails to fasten with the ampoule 100. The aperture 109 may have an intruding structure coupling with the cap. The material for the cap may be flexible to allow deformation to occur to form a liquid-tight fit. This alternation allows user to protect the ampoule 100 before use (e.g., during transportation).

In some implementations, a rib structure 107 is included along the longitudinal axis of the body 101 and in the plane defined by the opener 104. The rib structure 107 allows users to conveniently hold and grip the body 101 and avoid a slipping motion in the rotational direction. For example, a user can use three fingers (e.g. a middle finger, a ring finger and a pinky) to grip around the cylindrical portion of the body 101 and the other two figures (e.g. a thumb and an index finger) to hold the planar portion of the rib structure 107. This finger hold securely restricts motion of the tip 103 (e.g., to restrict the neck 102 and the body 101 from compliant motions such as rotation along with the opener 104). The user can then use the other hand's two fingers (e.g. a thumb and an index finger) to hold the opener 104 by the planar surface (e.g. pressing onto the surface, or to act on the rib portion), and apply a torque/moment to twist the opener 104 against the tip 103. Excessive deformation occurs when the torque exceeds a predetermined value so that the deformation can cause the twist coupler 105 to break away from the tip 103, revealing the aperture 109. Therefore, the body 101 includes an outer rim (i.e. the planar portion of the rib structure 107 and the opener 104) to provide torque support and an inner rim (i.e. the material between the tip 103 and the opener 104) to secure the twist coupler 105 to the outer rim.

As shown in FIG. 1, the opener 104 and the rib structure 1007 are separated below the bottom of the tip 103. This allows the tip 103 be completely inserted into a user's nostril without obstruction and/or causing discomfort. The rib structure 107 may be confined to a contour that avoids contact with a user's nostril when the tip 103 is fully inserted. Although the opener 104 and the rib structure 107 are shown separated near the bottom of the tip 103, other implementations also are contemplated in which the separation gap is placed at different locations, such as the shoulder of the body 101 or anywhere between the shoulder of the body 101 and the twist coupler 105. These examples, however, are non-limiting. Also, the opener 104 can have two steps of thicknesses: an outer rim for major torque support and an inner portion for securing the twist coupler 105 to the outer rim. In some implementations, the inner portion of the opener 104 can be of the same thickness as the rib structure 107, and does not contact the sidewalls of the tip 103. In the implementation illustrated in FIG. 1, the edges of the opener 104 are flush with the bottom of the tip 103 to facilitate the opening operation. The edges of the opener 104 is also flush or contiguous with the rib structure 107 of the body 101.

The bottom of the body 101 can include a dimension 108. The dimension 108 can include the diameter of the body 101 and a side extrusion step from the rib structure 107. To enhance portability and miniaturize the ampoule 100, the dimension 108 can be between about 15 mm and 25 mm (e.g., about 22 mm), and the extrusion portion of the rib structure 107 can be, for example, 1.5 mm. The diameter of the body 101 can be of any other values that, given certain length, can contain enough fluid for a one-time treatment, such as rinse, lavage, moisturize, etc. Various dimensions of the ampoule 100 also can exist. For example, the overall length 106 of the ampoule 100 can be between about 80 mm and 120 mm (e.g., 99 mm). The overall length 106 can be of any other value that fits within conventional purses, backpacks, briefcases, or other daily carry items.

FIG. 1B is a schematic top view of the ampoule 100, as shown in FIG 1A. In some implementations, the body 101, the tip 103, and the twist coupler 105 have circular cross section shape at various diameters. For example, the cross section of the twist coupler 105 may be a circular shape that has a diameter between about 4 and 10 mm (e.g., 6.35 mm). The maximum cross section of the tip 103 may have a diameter between about 10 and 20 mm (e.g., 15 mm). The cross section of the body 101 may have a diameter between about 15 and 25 mm (e.g., 22 mm) as the dimension 108. It can be seen from the top view that the opener 104 and the rib structure 107 align in the same plane that symmetrically divide the ampoule 100. Although the general cross section of the ampoule 1 is circular shape in this example, the cross section may be, in some implementations, a different practical shape that still provides a cylindrical body 101, such as elliptical shape for ease of applying pressure. In other examples useful for understanding the invention, the cross section may be a different practical shape, such as a triangular shape for packaging reasons, a diamond shape for both ease of applying pressure and packaging reasons, and/or a combination of different shapes at different cross section locations.

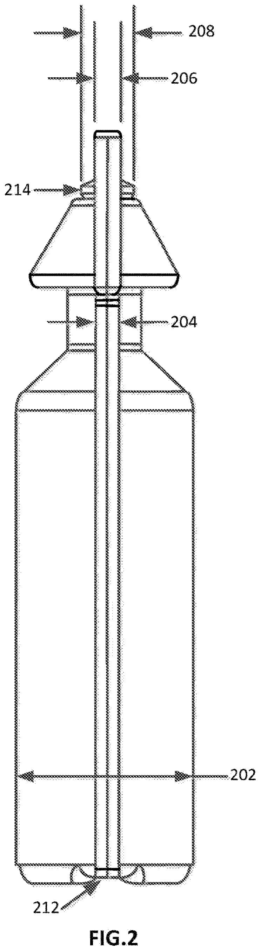

FIG 2 is a schematic side view of the ampoule 100, as shown in FIG. 1A. The side view shows additional structures of the ampoule 100. For example, the bottom of the body 101 is shaped for reinforcement and easy mold release that includes a concave surface 212 in the extrusion direction of the rib structure 107. Also, as shown in FIG. 2, the twist coupler 105 is attached to the tip 103 at a circular tangential portion 214 that acts as a plug or sealer for sealing the aperture 109. Further, in the example shown, the outer portion of the opener 104 is thicker than the rib structure 107. FIG. 2 further shows a dimension 202 to denote the diameter of the body 101, a dimension 204 to denote the thickness of the rib structure 107, a dimension 206 to denote the thickness of the opener 104, and a dimension 208 to denote the diameter of the twist coupler 105.

The circular tangential portion 214 connects the twist coupler 105 to the sealing aperture 109 by a cross section that has sufficient strength to maintain structural integrity during transportation (i.e. maintain shape under bending and tension loading conditions) and can be severed under shear stress in a twisting motion. In the example shown in FIG. 2, the circular tangential portion 214 has a low aspect ratio (e.g., height to diameter ratio is very small), allowing for very small moment arm for bending deformation. This shape profile enables resistance against bending failure modes. The circular tangential portion 214 is affixed to the opener 104 that allows for a large moment arm to be applied by user (at least about twice as large as the sealing cross section diameter). This allows for more material to be in contact at the sealing cross section between the circular tangential portion 214 and the aperture 109, better resisting tension or compression deformation.

To expose the aperture 109, a moment is applied to the opener 104 that is affixed to the twist coupler 105 by circumferential connection. The moment creates a shear stress concentrated at the circular tangential portion 214 while the connection between the twist coupler 105 and the opener 104 is under tension. Such as tearing apart a piece of paper is much easier than pulling apart a piece of paper, the cross section between the aperture 109 and the circular tangential portion 214 will fail or break before any other locations. This breaks apart the opener 104 and the tip 103 and exposes the aperture 109. The twist coupler 105 may include inner side walls that conform with but do not contact sidewalls of the tip 103.

In some implementations, the circular tangential portion 214 may have a donut-shape, a tire shape, or any other low aspect ratio cylindrical shapes that enable separation from the aperture 109 with shear stress. In some implementations, the circular tangential portion 214 may be of the same cross section shape as the aperture 109 and/or the tip 103.

The concave surface 212 at the bottom of the body 101 illustrated in FIG. 2 has multiple purposes, such as reinforcing the structural integrity of the body 101, enabling faster manufacturing process, allowing user to recognize the ampoule orientation, etc. In some implementations, the concave surface 212 creates a strengthening profile of the bottom of the body 101 by increasing the moment of inertia of the structure. This is the similar principle applied to most thin-walled bottles that use the shape instead of materials to achieve certain desired strength. The concave surface 212 also creates a strong local structure of the body 101 to withstand relatively large external loads. This may facilitate the manufacturing process when the body 101 is to be handled by various machines.

Various dimensions of the ampoule 100 are possible and illustrated in FIG. 2. For example, the diameter 202 of the body 101 can be in the range between 12 and 30 mm (e.g., about 20.5 mm). The diameter 202 can be of any other values that, given certain length, can contain enough fluid for a one-time treatment. In some implementations, the thickness 204 of the rib structure 107 can be in the range of 1 to 2 mm (e.g., about 1.4 mm). In some implementations, the thickness 204 can be of any other values so that, when loaded to twist open the ampoule 100, the rib structure 107 can maintain the original shape without excessive deformation.

In some implementations, the thickness 206 of the opener 104 can be in the range between 2 and 3 mm (e.g., about 2.4 mm). In some implementations, the thickness 206 can be at least 1 mm thicker than the thickness 204, or of any other values that gives the opener 204 enough structure integrity to twist open the coupler 105. In some implementations, the diameter 208 of the circular tangential portion 214 can be in the range between 3 and 8 mm (e.g., about 6.35 mm). The diameter 208 can be of any other values sufficient to provide a secure seal to the aperture 109.

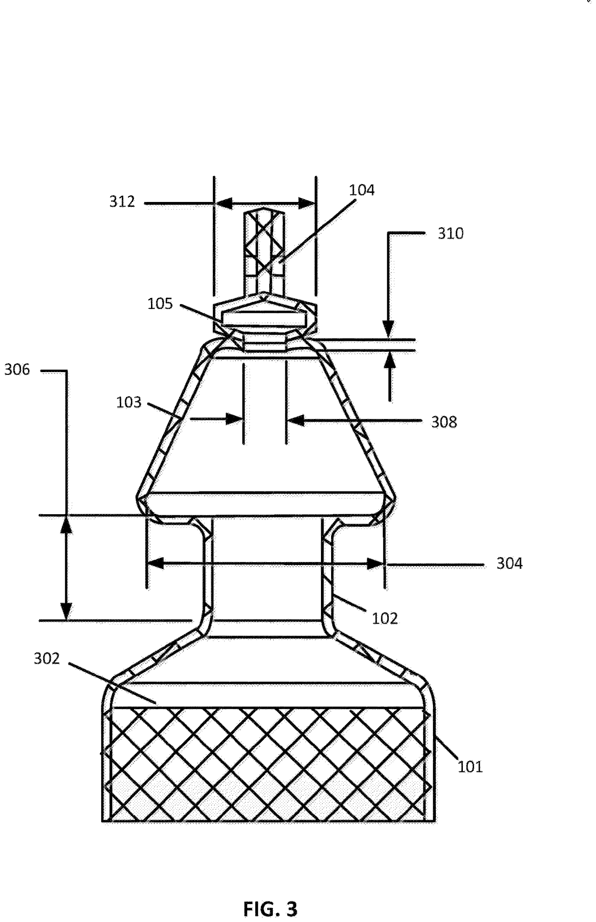

FIG. 3 is a schematic cross-sectional view of the ampoule 100. As shown in FIG. 3, portion of the body 101 is shown with a line 302 indicating the fill-up line for the fluid contained in the body 101. At about 20 ml fluid volume and about 20 mm body diameter, the line 302 can be about 66 mm from the bottom of the body 101. The position of the line 302 can change if a different fluid volume is to be filled and the body 101 is of a different diameter or size. The length 306 of the neck 102 can be in the range between 2 and 8 mm (e.g., about 6.4 mm), and can be of any other values that provides the rib structure 107 enough room for holding the ampoule 100.

In some implementations the rib structure 107 at the body 101 can be flush or contiguous with the rib structure 107 at the opener 104. The bottom diameter 304 of the tip 103 is in the range between 10 and 20 mm (e.g., 14.9 mm), while the top diameter 312 of the tip 103 is in the range between 3 and 8 mm (e.g., 6.35 mm). In some implementations, the top diameter 312 of the tip 103 may be the same value as the diameter 208. In some implementations, the diameter 308 of the aperture 109 can be in the range between 1.5 and 3.5 mm (e.g., 2.54 mm). In some implementations, the overall structure can be of a uniform thickness 310, which can be in the range between 0.3 and 0.8 mm (e.g., 0.65 mm).

As discussed above, the ampoule 100 can be made of a thermoplastic polymer, or thermoplastics. Most thermoplastics are high-molecular-weight polymers whose chains associate through weak Van der Waals forces (e.g. polyethylene); stronger dipole-dipole interactions and hydrogen bonding (e.g. nylon); or even stacking of aromatic rings (e.g. polystyrene). For example, the ampoule 100 can be made of acrylonitrile butadiene styrene, acrylic, celluloid, cellulose acetate, cyclic olefin copolymer, ethylene-vinyl acetate, ethylene vinyl alcohol, fluoroplastics, lonomers, Kydex, liquid crystal polymer, polyoxymethylyne, polyacrylates, polyacrylonitrile, polyamide, polyamide-imide, polyaryletherketone, polybutadiene, polybutylene, polybutylene terephthalate, polycaprolactone, polychlorotrifluoroethylene, polyethylene terephthalate, polycyclohexylene dimethylene terephthalate, polycarbonate, polyhydroxyalkanoates, polyketone, polyester, polyethylene, polyetheretherketone, polyetherketoneketone, polyetherimide, polyethersulfone, chlorinated polyethylene, polyimide, polylactic acid, polymethylpentene, polyphenylene oxide, polyphenylene sulfide, polyphthalamide, polypropylene, polystyrene, polysulfone, polytrimethylene terephthalate, polyurethane, polyvinyl acetate, polyvinyl chloride, polyvinylidene chloride, styrene-acrylonitrile, and/or a combination of these, or any other appropriate thermoplastics.

In some implementations, the ampoule 100 can be made of a thermosetting polymer, or thermoset. Thermoset is a polymer material that cures irreversibly through heat (generally above 200 °C (392 °F)), through a chemical reaction (two-part epoxy, for example), or irradiation such as electron beam processing. In some instances, the ampoule 100 can be made of vulcanized rubber, bakelite, duroplast, melamine resin, phenol formaldehyde, urea formaldehyde, melamine formaldehyde, polyester, epoxy, isoprene crosslinked with sulphur, neoprene, trihydroxymehylsilane, and/or a combination of these, or any other appropriate thermosetting polymers.

In some implementations, the ampoule 100 can be coated internally with a layer of epoxy resin to prevent reaction between the fluid and the body material. For example, if the ampoule 100 is coated with a layer of metal for light isolation or uses a metallic material for construction, then a layer of epoxy resin can provide isolation of the fluid and prevent undesired materials leaching into the liquid or solution contained in the body 101.

In some implementations, the ampoule 100 can be transparent overall, or in a portion such that remaining portion of fluid may be monitored. For example, the ampoule 100 may be made of a clear thermoplastic polymer, and/or a clear thermosetting polymer.

In some implementations, the ampoule 100 can use various materials for the body 101, the neck 102 and the tip 103. For example, the body 101 can use a thermoplastic polymer while the neck 102 and the tip 103 can use a thermosetting polymer. A variation of material in different parts of the ampoule 100 can improve durability, provide convenience to use, or enhance other characteristics of the ampoule 100 such as gripping.

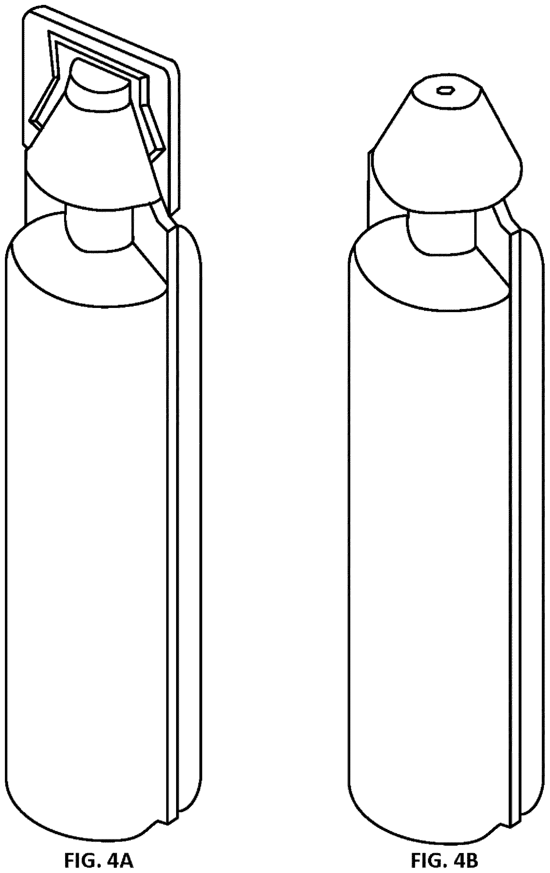

FIG. 4A is a one schematic view of the ampoule 100 before removing the opener 104, and FIG. 4B is another schematic view of the ampoule 100 after removing the opener 104. As shown in FIG. 4A, the opener 104 and the twist coupler 105 are attached to the tip 103. The rib structure 107 allows users to conveniently hold and grip the body 101 and avoid slipping motion in the rotational direction. Upon removing the opener 104, the aperture 109 is revealed.

In some implementations, a user should be in a upright position before using the opened ampoule 100, which is shown in FIG. 4B. The head of the user can be tilted to one side slightly. After placing the tip 103 into one nostril, the user can press gently to dispense a few drops or a small quantity for moisture or squeeze to expel a larger quantity for nasal irrigation. After one use, the whole ampoule 100 can be discarded, along with unused solution. The ampoule 100 can be used to contain fluid that is a drug-free, preservative-free, sterile nasal saline solution. The solution can sooth and moisturize dry and congested noses for babies, children and adults. In the 20 ml volume implementation shown in FIG. 3, the ampoule 100 is convenient for home, nursery, playground, school, air travel, hotel room and hospital use. A few drops of saline can moisturize, while squeezing a larger quantity can deliver a gentle low pressure low volume nasal rinse directly into the nostril for stronger results.

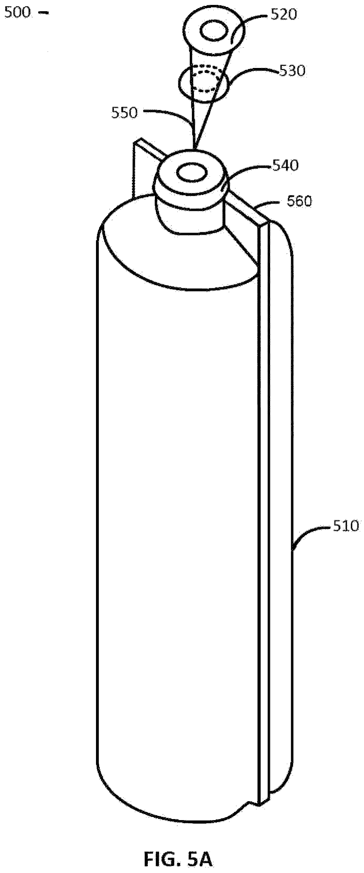

FIG. 5A is a schematic view of a second ampoule example useful for understanding the invention. In this example, the second ampoule 500 includes a body 510 and a cap 550. The body 510 can be a fluid container (e.g. a bottle, can or other container) that securely stores fluid and allows users to apply pressure (e.g. to the container) to expel the stored fluid. For example, the body 510 can be made of thermoplastic polymers, thermosetting polymers, or any other appropriate materials that allows for deformation in order to pressurize the bottle for fluid release. In some examples useful for understanding the invention, the ampoule 500 can be pressurized for maintaining shape during transportation. In some examples useful for understanding the invention, the body 510 is a cylindrical shape of a uniform diameter. In some examples useful for understanding the invention, the diameter can vary along its longitudinal axis, for example, a tapered shape, a curved shape, a diamond shape, or other shapes. The body 510 can be a thin-walled structure of uniform thickness and/or variable thickness for functional requirements. For example, to facilitate deformation, some locations on the body 510 can be thinner than the rest. As another example, other locations on the body 510 can be thicker for structural reinforcement, such as a portion at or near the bottom of the ampoule 500. Grooves or depressions can be included in the body to facilitate gripping by the human hand.

The body 510 may include a rib structure 560 to facilitate holding or gripping of the body 510 to avoid slipping motion. The rib structure 560 may be flush or contiguous with edges of the opener 520, or may be only extended to a functional portion around the body 510. The rib structure 560 may extend from a bottom portion of the tip to a bottom portion of the body 510.

As shown in FIG 5A, the cap 550 is a conical needle head structure for securely sealing the ampoule body 510 and allowing for reuse. The cap 550 includes two structural features besides the needle head shape: a pulling support 520 and a sealing support 530. The pulling support 520 enables user to apply a tension force to separate the cap 550 from the body 510. The pulling support 520 may be a sudden increase in diameter of the conical shape of the cap 550. This resulting step structure allows user's fingers to engage with the cap 550. The sealing support 530 is an extruding structure near the middle location of the cap 550. The sealing support 530 engages with the sealing end 540 at the tip of the body 510. The sealing end 540 may be a donut, a tire or other inner grooved shape that couples with the sealing support 530 under a predetermined stress that seals the aperture of the body 510.

In some examples useful for understanding the invention, the ampoule 500 can be made of a thermoplastic polymer, or thermoplastics. Most thermoplastics are high-molecular-weight polymers whose chains associate through weak Van der Waals forces (e.g. polyethylene); stronger dipole-dipole interactions and hydrogen bonding (e.g. nylon); or even stacking of aromatic rings (e.g. polystyrene). For example, the ampoule 500 can be made of acrylonitrile butadiene styrene, acrylic, celluloid, cellulose acetate, cyclic olefin copolymer, ethylene-vinyl acetate, ethylene vinyl alcohol, fluoroplastics, lonomers, Kydex, liquid crystal polymer, polyoxymethylyne, polyacrylates, polyacrylonitrile, polyamide, polyamide-imide, polyaryletherketone, polybutadiene, polybutylene, polybutylene terephthalate, polycaprolactone, polychlorotrifluoroethylene, polyethylene terephthalate, polycyclohexylene dimethylene terephthalate, polycarbonate, polyhydroxyalkanoates, polyketone, polyester, polyethylene, polyetheretherketone, polyetherketoneketone, polyetherimide, polyethersulfone, chlorinated polyethylene, polyimide, polylactic acid, polymethylpentene, polyphenylene oxide, polyphenylene sulfide, polyphthalamide, polypropylene, polystyrene, polysulfone, polytrimethylene terephthalate, polyurethane, polyvinyl acetate, polyvinyl chloride, polyvinylidene chloride, styrene-acrylonitrile, and/or a combination of these, or any other appropriate thermoplastics.

In some examples useful for understanding the invention, the ampoule 500 can be made of a thermosetting polymer, or thermoset. Thermoset is a polymer material that cures irreversibly through heat (generally above 200 °C (392 °F)), through a chemical reaction (two-part epoxy, for example), or irradiation such as electron beam processing. In some instances, the ampoule 500 can be made of vulcanized rubber, bakelite, duroplast, melamine resin, phenol formaldehyde, urea formaldehyde, melamine formaldehyde, polyester, epoxy, isoprene crosslinked with sulphur, neoprene, trihydroxymehylsilane, and/or a combination of these, or any other appropriate thermosetting polymers.

In some examples useful for understanding the invention, the ampoule 500 can be coated internally with a layer of epoxy resin to prevent reaction between the fluid and the body material. For example, if the ampoule 500 is coated with a layer of metal for light isolation or uses a metallic material for construction, then a layer of epoxy resin can provide isolation of the fluid and prevent undesired materials leaching into the liquid or solution contained in the body 510.

In some examples useful for understanding the invention, the ampoule 500 can be transparent overall, or in a portion such that remaining portion of fluid may be monitored. For example, the ampoule 500 may be made of a clear thermoplastic polymer, and/or a clear thermosetting polymer.

In some examples a useful for understanding the invention, the sealing support 530 may be made of a material different from that of the body 510, such as metal, for preferred elastic modulus and reliability. The sealing support 530 may be made in a shape that conforms to the inner chamber of the sealing end 540. The shape of the sealing support 530 may experience substantial elastic deformation during the coupling and/or decoupling process with the sealing end 540. In some cases, the sealing support 530 may be made of a foil of stainless steel forming a donut shape to couple with the sealing end 540.

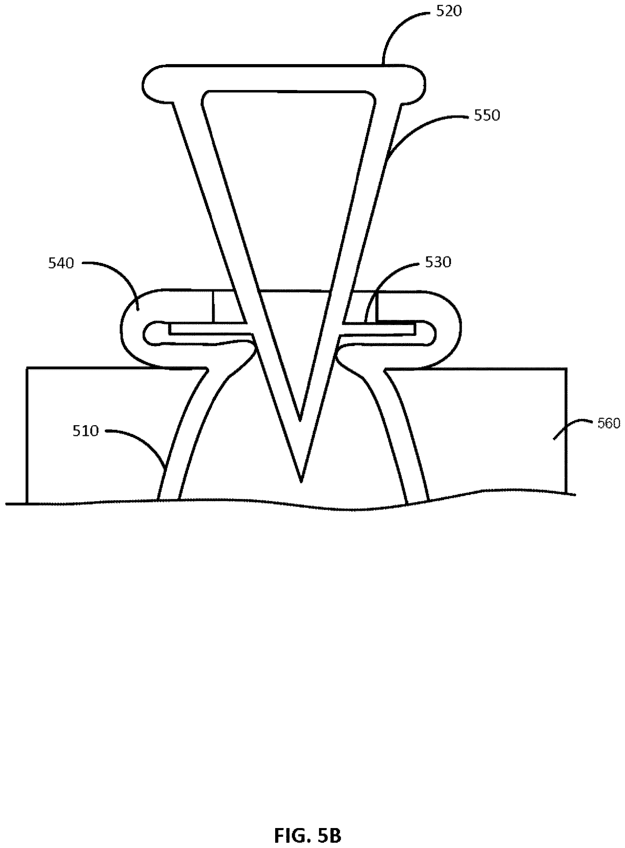

FIG. 5B is a schematic cross-sectional view along the longitudinal axis of the second ampoule example that is useful for understanding the invention. This cross-sectional view shows details about the engagement between the cap 550 and the body 510. The sealing end 540 at the tip of the body 510 may have two inner edges that conform to the tapered needle cap 550. The upper edge of the sealing end 540 may provide a sealing force closing the cap 550 towards the lower edge of the sealing end 540. The cap 550 can be a thin-walled structure of approximate uniform thickness. The cap 550 may experience substantial elastic deformation during the coupling and/or decoupling process with the body 510 at the sealing end 540.

A number of embodiments of the disclosure have been described. Nevertheless, it will be understood that various modifications can be made within the scope of the invention as defined by the appended claims. For example, instead of attenuating a fast stream of liquid into a gentle flow, a mist exiting the actuator can be transformed into a gentle cleansing stream of fluid.

## Claims

1. A dispensing device (100) for releasing a fluid into a nostril of a human body comprising:
a body (101), being a cylindrical fluid container extending along a longitudinal axis, which allows users to apply pressure to the container to expel the stored fluid;
a cylindrical neck (102) extending along said longitudinal axis and coupled to the body (101);
a tip (103) coupled to the neck (102), the tip having an aperture (109) thereon through which the fluid is released; and
a sealing device coupled to the tip and configured to seal the aperture, where the sealing device permanently unseals the aperture upon decoupling the sealing device from the tip;
wherein the tip (103) includes a tapered surface that permits the tip to conform to nostrils of different sizes, the exterior of the tip (103) being tapered outwardly from a wide portion up near the bottom of the tip (103) to a narrow portion near the top of the tip, and the tip being sized to prevent the wide portion from extending all the way into the user's nostril;
wherein the bottom diameter (304) of the tip (103) is between 10 and 20 millimeters, and the top diameter (312) of the tip (103) is between 3 and 8 millimeters; and
wherein the sealing device includes an opener (104) having a twist coupler (105) to facilitate twisting motion for removing the sealing device to unseal the aperture (109).

2. The dispensing device of claim 1, wherein the opener (104) is a planar holding structure to accommodate application of moment or torque to the twist coupler (105).

3. The dispensing device of claim 1, wherein the opener (104) includes an outer rim to provide torque support and an inner rim to secure the twist coupler to the outer rim.

4. The dispensing device of claim 1, wherein the sealing device includes a sealer and the twist coupler (105) is coupled to the tip (103) through the sealer.

5. The dispensing device of claim 4, wherein the body (101) includes a rib structure to facilitate holding or gripping of the body to avoid a slipping motion generated when the body is rotated in an opposite direction with respect to the sealing device.

6. The dispensing device of claim 5, wherein the rib structure (107) is flush or contiguous with edges of the opener.

7. The dispensing device of claim 6, wherein the rib structure (107) extends from a bottom portion of the tip to a bottom portion of the body.

8. The dispensing device of claim 7, wherein a bottom surface of the opener (104) aligns with the bottom portion (111) of the tip.

9. The dispensing device of claim 1, wherein the twist coupler (105) includes inner sidewalls that conform with but do not contact sidewalls of the tip.

10. The dispensing device of claim 1, wherein the neck (102) has a diameter (204) smaller than a diameter (202) of the body (101) to facilitate a dispensing speed of the solution.

11. The dispensing device of claim 1, wherein the tip (103) is conically shaped with a convex curved surface tapered from a surface on which the aperture is formed to a bottom portion (111) of the tip.

## Patentansprüche

1. Abgabevorrichtung (100) zum Freisetzen eines Fluids in ein Nasenloch eines menschlichen Körpers, umfassend:
einen Körper (101), bei dem es sich um einen zylindrischen Flüssigkeitsbehälter handelt, der sich entlang einer Längsachse erstreckt und der es Benutzern ermöglicht, Druck auf den Behälter auszuüben, um die gespeicherte Flüssigkeit auszustoßen;
einen zylindrischen Hals (102), der sich entlang der Längsachse erstreckt und mit dem Körper (101) verbunden ist;
eine Spitze (103), die mit dem Hals (102) verbunden ist, wobei die Spitze eine Öffnung (109) aufweist, durch die die Flüssigkeit freigesetzt wird; und
eine Abdichtungsvorrichtung, die mit der Spitze verbunden und so konfiguriert ist, dass sie die Öffnung abdichtet, wobei die Abdichtungsvorrichtung die Öffnung beim Abkoppeln der Abdichtungsvorrichtung von der Spitze dauerhaft entsiegelt,
wobei die Spitze (103) eine sich verjüngende Oberfläche aufweist, die es ermöglicht, dass sich die Spitze an Nasenlöcher unterschiedlicher Größe anpasst, wobei das Äußere der Spitze (103) von einem breiten Abschnitt oben in der Nähe des Bodens der Spitze (103) zu einem schmalen Abschnitt in der Nähe des oberen Endes der Spitze nach außen hin verjüngt ist und die Spitze so bemessen ist, dass sie verhindert, dass sich der breite Abschnitt ganz in das Nasenloch des Benutzers hinein erstreckt,
wobei der untere Durchmesser (304) der Spitze (103) zwischen 10 und 20 Millimetern und der obere Durchmesser (312) der Spitze (103) zwischen 3 und 8 Millimetern beträgt, und
wobei die Abdichtungsvorrichtung einen Öffner (104) mit einer Drehkupplung (105) umfasst, um eine Drehbewegung zum Entfernen der Abdichtungsvorrichtung zu erleichtern, um die Öffnung (109) zu entsiegeln.

2. Abgabevorrichtung nach Anspruch 1, wobei der Öffner (104) eine ebene Haltestruktur ist, um das Aufbringen eines Moments oder Drehmoments auf die Drehkupplung (105) zu ermöglichen.

3. Abgabevorrichtung nach Anspruch 1, wobei der Öffner (104) einen äußeren Rand zur Drehmomentabstützung und einen inneren Rand zur Befestigung der Drehkupplung an dem äußeren Rand aufweist.

4. Abgabevorrichtung nach Anspruch 1, wobei die Versiegelungsvorrichtung eine Versiegelung umfasst und die Drehkupplung (105) durch die Versiegelung mit der Spitze (103) verbunden ist.

5. Abgabevorrichtung nach Anspruch 4, wobei der Körper (101) eine Rippenstruktur aufweist, um das Halten oder Greifen des Körpers zu erleichtern, um eine Gleitbewegung zu vermeiden, die erzeugt wird, wenn der Körper in einer entgegengesetzten Richtung in Bezug auf die Versiegelungsvorrichtung gedreht wird.

6. Abgabevorrichtung nach Anspruch 5, wobei die Rippenstruktur (107) mit den Kanten des Öffners bündig ist oder an diese angrenzt.

7. Abgabevorrichtung nach Anspruch 6, wobei sich die Rippenstruktur (107) von einem unteren Abschnitt der Spitze zu einem unteren Abschnitt des Körpers erstreckt.

8. Abgabevorrichtung nach Anspruch 7, wobei eine untere Fläche des Öffners (104) mit dem unteren Abschnitt (111) der Spitze ausgerichtet ist.

9. Abgabevorrichtung nach Anspruch 1, wobei die Drehkupplung (105) innere Seitenwände aufweist, die mit den Seitenwänden der Spitze konform sind, diese aber nicht berühren.

10. Abgabevorrichtung nach Anspruch 1, wobei der Hals (102) einen Durchmesser (204) aufweist, der kleiner als ein Durchmesser (202) des Körpers (101) ist, um eine Abgabegeschwindigkeit der Lösung zu erleichtern.

11. Abgabevorrichtung nach Anspruch 1, wobei die Spitze (103) konisch geformt ist mit einer konvex gekrümmten Oberfläche, die sich von einer Oberfläche, an der die Öffnung ausgebildet ist, zu einem unteren Abschnitt (111) der Spitze verjüngt.

## Revendications

1. Dispositif de distribution (100) pour distribuer un fluide dans une narine d'un corps humain, comprenant :
un corps (101), qui est un récipient de fluide cylindrique s'étendant le long d'un axe longitudinal, qui permet à des utilisateurs d'appliquer une pression au récipient pour expulser le fluide stocké ;
un col cylindrique (102) s'étendant le long dudit axe longitudinal et couplé au corps (101) ;
un embout (103) couplée au col (102), l'embout présentant une ouverture (109) à travers laquelle le fluide est libéré ; et
un dispositif d'étanchéité couplé à l'embout et configuré pour rendre l'ouverture étanche, dans lequel le dispositif d'étanchéité libère l'étanchéité de l'ouverture de manière permanente lors du découplage du dispositif d'étanchéité à partir de l'embout ;
dans lequel l'embout (103) comprend une surface effilée qui permet à l'embout de se conformer à des narines de différentes tailles, l'extérieur de l'embout (103) étant effilé vers l'extérieur à partir d'une partie large vers le haut près du bas de l'embout (103) vers une partie étroite près du haut de l'embout, et l'embout étant dimensionné pour empêcher la partie large de s'étendre jusque dans la narine de l'utilisateur ;
dans lequel le diamètre inférieur (304) de l'embout (103) est compris entre 10 et 20 millimètres, et le diamètre supérieur (312) de l'embout (103) est compris entre 3 et 8 millimètres ; et
dans lequel le dispositif d'étanchéité comprend un dispositif d'ouverture (104) présentant un coupleur de torsion (105) pour faciliter un mouvement de torsion pour retirer le dispositif d'étanchéité afin de libérer l'étanchéité de l'ouverture (109).

2. Dispositif de distribution selon la revendication 1, dans lequel le dispositif d'ouverture (104) est une structure de maintien plane pour permettre l'application d'un moment ou d'un couple au coupleur de torsion (105).

3. Dispositif de distribution selon la revendication 1, dans lequel le dispositif d'ouverture (104) comprend un rebord extérieur pour fournir un support de couple et un rebord intérieur pour fixer le coupleur de torsion au rebord extérieur.

4. Dispositif de distribution selon la revendication 1, dans lequel le dispositif d'étanchéité comprend un organe d'étanchéité et le coupleur de torsion (105) est couplé à l'embout (103) par l'intermédiaire de l'organe d'étanchéité.

5. Dispositif de distribution selon la revendication 4, dans lequel le corps (101) comprend une structure de nervure pour faciliter le maintien ou la préhension du corps afin d'éviter un mouvement de glissement généré lorsque le corps est tourné dans une direction opposée par rapport au dispositif d'étanchéité.

6. Dispositif de distribution selon la revendication 5, dans lequel la structure de nervure (107) est affleurante ou contiguë aux bords de du dispositif d'ouverture.

7. Dispositif de distribution selon la revendication 6, dans lequel la structure de nervure (107) s'étend à partir d'une partie inférieure de l'embout jusqu'à une partie inférieure du corps.

8. Dispositif de distribution selon la revendication 7, dans lequel une surface inférieure du dispositif d'ouverture (104) s'aligne avec la partie inférieure (111) de l'embout.

9. Dispositif de distribution selon la revendication 1, dans lequel le coupleur de torsion (105) comprend des parois latérales intérieures qui se conforment aux parois latérales de l'embout mais ne viennent pas en contact avec celles-ci.

10. Dispositif de distribution selon la revendication 1, dans lequel le col (102) présente un diamètre (204) inférieur à un diamètre (202) du corps (101) pour faciliter une vitesse de distribution de la solution.

11. Dispositif de distribution selon la revendication 1, dans lequel l'embout (103) est de forme conique avec une surface incurvée convexe effilée à partir d'une surface sur laquelle l'ouverture est formée jusqu'à une partie inférieure (111) de l'embout.
